# FASCICULE DE BREVET EUROPEEN

(11) **EP 0 680 465 B1**
(45) Date de publication et mention de la délivrance du brevet: **22.04.1998**
(21) Numéro de dépôt: 94905140.3
(22) Date de dépôt: 21.01.1994
(51) Int. Cl.: C07C 231/02, C07C 233/36

(54) **PROCEDE DE PREPARATION D'UN MONOAMIDE ALPHA-AMINO OMEGA-ESTER**
VERFAHREN ZUR HERSTELLUNG VON ALPHA-AMINO OMEGA-ESTER-MONOAMID
METHOD FOR PREPARING AN ALPHA-AMINO OMEGA-ESTER MONOAMIDE

(30) Priorité: 25.01.1993 FR 9300909
(43) Date de publication de la demande: 08.11.1995
(73) Titulaire: RHODIA CHIMIE, 92408 Courbevoie Cédex (FR)
(72) Inventeur: JOUFFRET, Frédéric, F-69340 Francheville (FR); MADEC, Pierre-Jean, F-45000 Orléans (FR)
(74) Mandataire: Esson, Jean-Pierre
(86) Numéro de dépôt international: FR9400073
(87) Numéro de publication internationale: WO9417030

(56) Documents cités:
- EP-A- 0 207 539
- GB-A- 461 237
- ANNALES DE CHIMIE - SCIENCE DES MATERIAUX vol. 12, no. 6 , 1951 , PARIS FR pages 53 - 103 S. Z. SZEWCZYK 'Préparation des acides N(aminoalcoyle) aminoundécanoiques; des NN'[di.omega.(carboxyldécyl)] polyéthylènediamines et des polymères qui en dérivant'
- MAKROMOLEKULARE CHEMIE, MACROMOLECULAR CHEMISTRY AND PHYSICS vol. 23 , 1957 , BASEL CH pages 85 - 118 H. ZAHN ET AL. 'Über lineare Oligamide der Adipinsäure mit Hexamethylendiamin'

## Description

L'invention concerne un procédé de préparation d'un monoamide α-amino ω-ester appelé monoamide ester.

Elle concerne plus particulièrement un procédé de préparation directe d'un monoamide ester.

Les polyamides sont des matières synthétiques connues et découvertes au début du XXème siècle. Ainsi le polyadipamide d'hexaméthylène plus connu sous le nom de PA 6.6 ou Nylon 6.6 a été synthétisé pour la première fois par W.H. Carothers en 1936.

Ces polyamides sont généralement utilisés industriellement pour la production de fibres synthétiques dans des applications comme le textile, ou comme fibres de renforcement des différents matériaux comme élastomères, autres matières plastiques, ciments etc...

Ils sont également utilisés comme résine matricielle pour la fabrication de pièces moulées. Dans cette application, ils peuvent contenir de nombreuses charges améliorant notamment leurs propriétés mécaniques, électriques ou de résistance au feu.

Il existe deux grands procédés de fabrication d'un polyamide. Dans le premier, le polyamide est obtenu par homopolymérisation d'un aminoacide, tel que, par exemple, le caprolactame qui conduit à un polyamide appelé PA 6. Les polyamides obtenus selon ce premier procédé sont généralement identifiés par les lettres PA suivi d'un seul nombre.

Dans le second procédé, le polyamide est obtenu par réaction entre une diamine et un diacide. Les polyamides les plus connus fabriqués selon ce second procédé, sont le PA 6.6. obtenu par action de l'hexaméthylène diamine sur l'acide adipique, le PA 6.10 obtenu par action de l'hexaméthylène diamine sur l'acide sébacique. Une autre classe de polyamides obtenus selon ce second procédé comprend les polyamides aromatiques ou semi-aromatiques qui présentent des propriétés mécaniques et de tenue thermique élevées. Comme polyamides appartenant à cette classe, on peut citer les polytéréphtalate d'hexaméthylène; polyisophtalate d'hexaméthylène ou leurs copolymères.

Le procédé de polymérisation utilisant cette deuxième catégorie de réaction requiert un contrôle de la stoechiométrie entre la diamine et le diacide de départ pour obtenir des polymères de haut poids moléculaire. Cette maîtrise est souvent difficile à réaliser car il se produit généralement des évaporations de l'un des réactifs, nécessitant l'addition d'un excès de ce réactif dans le procédé et un contrôle important des paramètres de conduite du procédé. De ce fait, l'obtention de polymère à très haut poids moléculaire ou de viscosité élevée nécessite souvent la réalisation d'une post condensation solide, requérant une forte consommation d'énergie et pouvant générer des dégradations du polymère.

Par ailleurs, le monoamide α-amino ω-ester dérivant par exemple de l'acide adipique et de l'hexaméthylène diamine est connu, notamment dans l'article publié par ZAHN et al. dans Makromol. Chem. 36 (1956) 85-119 . Ce produit a été identifié dans le mélange obtenu suite à la réaction entre l'hexaméthylène diamine et l'adipate de diéthyle et reprise du milieu réactionnel par un solvant. Aucun document ne décrit un procédé permettant de synthétiser ce composé de manière spécifique et sélective soit directement soit par un procédé de séparation et de purification.

La présente invention a notamment pour but de remédier à ces inconvénients en proposant un procédé de préparation d'un monoamide α-amino ω-ester permettant d'obtenir directement et de manière sélective et spécifique ce composéαω.

A cet effet, l'invention propose un procédé de préparation d'un monoamide α-amino ω-ester de formule générale (IV)

H₂N - B - NH - CO - A - COOR₁ (IV)

dans laquelle
- B représente un reste de diamine et
- A représente un reste d'un diacide
- R₁ représente un groupement alkyle comprenant de 1 à 6 atomes de carbone,
caractérisé en ce qu'il consiste :
- à mélanger une diamine de formule :

   H₂N - B - NH₂ (II)

   et un diester de formule :

   R₁OOC - A - COOR₁ (III)

   dans un rapport molaire diamine/diester compris entre 0,8 et 1,2,avantageusement entre 1 et 1,1,
- à maintenir ce mélange en phase liquide à une température inférieure à la température de fusion ou de cristallisation du monoamide α-amino ω-ester formé, jusqu'à précipitation dudit monoamide α-amino ω-ester et à récupérer le précipité formé.

Le composé ainsi obtenu permet la préparation d'un polymère comprenant des unités de récurrence de formule I suivante :

-[-CO - A - CO - NH - B - NH -]ₙ- (I)

dans laquelle A et B différents ou identiques sont des restes de diacides et de diamines et représentent des groupements hydrocarbonés aliphatiques, aromatiques, aromatique/aliphatiques ou aliphatique/aromatiques,
n est un nombre compris entre 20 et 700

Ce procédé consiste à réaliser l'homopolymérisation du monoamide α-amino ω-ester de formule générale (IV) par chauffage d'au moins un monoamide α-amino ω-ester à une température comprise entre 170°C et 400°C et à maintenir la masse réactionnelle fondue, sous agitation, à une température comprise entre 170°C et 400°C jusqu'à obtention d'une masse réactionnelle présentant une viscosité inhérente Iv désirée.

Ce procédé de fabrication d'un polymère contenant des unités de récurrence de formule générale (I) consiste à réaliser dans une étape 1 la préparation d'un monoamide α-amino ω-ester, selon le procédé qui forme le premier objet de l'invention, et de réaliser dans une étape 2, après avoir isolé et éventuellement purifié par lavage par exemple, le produit fabriqué à l'étape 1, l'homopolymérisation du monoamide α-amino ω-ester

Pour éviter les répétitions et pour une plus grande clarté, I' objet de l'invention est décrit comme étape 1 et étape 2 de l'exposé du procédé de fabrication d'un polymère.

Conformément à l'invention, le procédé de fabrication d'un polymère comprenant des unités de récurrence de formule (I) comprend deux étapes successives.

Dans une première étape, appelée étape 1, on fait réagir une diamine de formule (II) suivante :

H₂N - B - NH₂ (II)

avec un diester d'un acide de formule générale (III)

R₁OOC - A - COOR₁ (III)

dans laquelle R₁ identiques ou différents représentent un groupement hydrocarboné comprenant de 1 à 6 atomes de carbone, de préférence un groupement alkyle, tel que par exemple les radicaux méthyl, éthyl.

Selon une autre caractéristique de l'invention, la diamine et le diester d'acide sont mis en oeuvre selon un rapport stoechiométrique égal à 1 ou très voisin, de préférence compris entre 0,8 et 1,2, avantageusement entre 1 et 1,1.

Le précipité obtenu lors de cette étape 1 est un monoamide α-amino ω-ester de formule générale (IV)

H₂N-B-NH-CO-A-COOR₁ (IV)

sa structure a été confirmée par des analyses spectrales telles que RMN du ¹³C.

Ce produit peut être purifié, notamment pour éliminer les produits de départ qui n'ont pas réagi, par lavage à l'eau, par exemple.

Le produit ainsi obtenu est stable au stockage à température ambiante, sous atmosphère inerte ou sous air.

L'étape 1 du procédé de l'invention permet donc d'obtenir un oligomère d'un polyamide présentant un rapport stoechiométrique parfait entre les deux monomères de départ.

En outre, au cours de cette étape 1, les pertes de l'un des monomères, par exemple par évaporation, sont minimes car elle est réalisée à une température très basse, c'est-à-dire à une température légèrement supérieure à la température de fusion de l'un des monomères de départ, par exemple à la température de fusion du monomère le moins fusible. Avantageusement, cette température sera supérieure de 1 à 30°C à la température de fusion.

Dans un mode de réalisation préféré, l'étape 1 est réalisée à une température à laquelle au moins l'un des produits de départ est à l'état fondu, l'autre étant soit sous forme fondue, solubilisée ou dispersée, cette température étant inférieure à la température de fusion ou de cristallisation du monoamide α-amino ω-ester. Ainsi, ce dernier précipite dès sa formation .

Dans un autre mode de réalisation de l'étape 1 du procédé de l'invention, les produits de départ sont dissous dans un solvant mutuel dans lequel le monoamide α-amino ω-ester est insoluble. Ainsi, comme dans le premier mode de réalisation, le monoamide α-amino ω-ester précipitera dès sa formation.

Selon une autre caractéristique de l'invention, l'étape 1 est réalisée en présence d'un composé présentant une activité catalytique pour la réaction d'aminolyse entre la diamine et le diester d'acide. Ce composé comprend des groupements électrophiles et/ou nucléophiles.

Les composés convenables sont notamment les composés comprenant un groupement basique et/ou groupement acide.

Ces composés peuvent être ajoutés dans le milieu réactionnel dans des quantités en poids comprises entre 0,1 % et 10 % par rapport au mélange diamine/diester.

On peut citer comme composés à action catalytique convenables pour l'invention, les acides forts, bases fortes, les alkoxylates d'alcalins tel que le méthoxylate de sodium, les aryloxylates d'alcalins, les composés aromatiques comprenant une fonction phénol, tels que le phénol, le méta ou l'ortho-crésol, l'imidazole, les composés organométalliques tels que, par exemple, les composés organiques du titane ou du zirconium.

Parmi les acides forts,les composés préférés de l'invention sont les acides contenant du phosphore et leurs dérivés .

D'autres composés comprenant des groupements N-hétérocycliques tels que le pyrazole et ses dérivés, 8-hydroxyquinoléine, peuvent également être utilisés.

Cette liste est donnée uniquement à titre d'exemple et n'est pas limitative. En effet, tout catalyseur de la réaction d'aminolyse peut convenir pour l'invention.

Toutefois, dans un mode de réalisation préféré de l'invention, les catalyseurs préférés de l'invention sont ceux qui pourront être éliminés avant le début de la réaction d'homopolymérisation du monoamide α-amino ω-ester dans l'étape 2, c'est-à-dire, de préférence avant la température de fusion dudit monoamide α-amino ω-ester.

Cette élimination du catalyseur peut être réalisée soit lors du chauffage du monoamide α-amino ω-ester à l'étape 2 du procédé de polymérisation, par simple évaporation, soit par extraction par exemple par lavage à l'eau du précipité obtenu à l'étape 1.

Cette élimination du composé catalytiquement actif n'est pas une condition obligatoire du procédé de l'invention. Ainsi, pour certains catalyseurs il est intéressant de les conserver au cours de l'étape 2 car ils peuvent favoriser l'homopolymérisation du composé de formule (IV) :

NH₂ - B - NH - CO - A - CO - OR₁ (IV)

Cette seconde étape du procédé de polymérisation consiste à réaliser l'homopolymérisation du monoamide α-amino ω-ester de formule (IV) par chauffage de celui-ci à une température comprise entre 170°C et 400°C, et à maintenir le milieu à une température comprise entre 170°C et 400°C , pendant le temps nécessaire pour obtenir un polymère de masse moléculaire élevée. L'évolution de la masse moléculaire du polymère est contrôlée par la mesure d'uneViscosité inhérente du milieu réactionnel (Iv) selon une méthode décrite ci-dessous.Cette gamme de températures peut être différente et est variable selon la nature du polymère à fabriquer.

Le monoamide α-amino ω-ester est avantageusement fondu, la polymérisation étant réalisée en milieu fondu . La masse réactionnelle fondue est éventuellement agitée .

Selon une autre caractéristique, le maintien en température de la masse réactionnelle est réalisée sous une pression au plus égale à la pression atmosphérique.

Dans un mode de réalisation, le chauffage du monoamide-ester est réalisé sous reflux jusqu'à une température comprise entre 170°C et 400°C. Comme indiqué ci-dessus cette gamme de températures peut être différente et est variable selon la nature du polymère à fabriquer.

Au cours de ce chauffage, les produits très volatils comme les catalyseurs utilisés à l'étape 1 et l'alcool formé lors de l'homopolymérisation sont éliminés.

Après cette montée en température, la masse réactionnelle est maintenue à une température avantageusement supérieure à 170°C, sous pression atmosphérique. La polymérisation est avantageusement terminée en abaissant la pression au-dessus de la masse réactionnelle à une valeur pouvant se situer avantageusement entre 10² et 10⁵ Pa, la température pouvant être légèrement augmentée.

Quand le milieu réactionnel a atteint la viscosité désirée, il est refroidi.

Dans un autre mode de réalisation, le monoamide α-amino ω-ester de formule (IV) est directement chauffé à une température avantageusement comprise entre 170°C et 400°C, la pression au-dessus de la masse réactionnelle étant réduite pendant la période de chauffage par exemple, au plus tard quand le monoamide α-amino ω-ester est fondu. L'intervalle de températures données ci-dessus n'est qu'indicatif et peut varier selon la nature du polymère à synthétiser comme indiqué pour le premier mode de réalisation de l'étape 2.

La masse réactionnelle est maintenue à cette température pendant une durée suffisante pour obtenir un polymère ayant les caractéristiques moléculaires et de viscosité souhaitées. Le polymère ainsi obtenu est ensuite refroidi.

Le procédé de polymérisation permet ainsi d'obtenir un polyamide dont les analyses spectrales démontrent qu'il est tout à fait comparable à celui obtenu par polymérisation à partir d'un sel entre un diacide et une diamine, sel généralement appelé "sel Nylon" quand le polyamide est un polyadipate d'hexaméthylène.

Par ailleurs, comme les exemples ci-dessous l'illustreront, la cinétique de polymérisation du monoamide α-amino ω-ester est très rapide. En outre, il est possible d'atteindre des poids moléculaires et viscosité élevés, directement par le procédé de polymérisation en milieu fondu ou solide.

Les analyses des polymères obtenus montrent que leur dégradation et la présence de composés produits par des réactions secondaires sont très limitées et au plus égales à celles présentes dans les procédés connus de polymérisation à partir de diacides et de diamines.

Le procédé de l'invention peut être mis en oeuvre pour la production de nombreux polyamides. En effet, le procédé est applicable dès qu'il est possible de fabriquer de manière sélective un monoamide α-amino ω-ester, c'est-à-dire que ce dernier peut être obtenu par précipitation, dès sa formation, du milieu réactionnel contenant les monomères de départ, comme dans l'étape 1 décrite ci-dessus formant l'objet de l'invention.

Ainsi, le procédé de l'invention est applicable pour fabriquer des polymères comprenant des unités de récurrence de formule (I) dans laquelle le radical A représente un groupement hydrocarboné choisi dans le groupe comprenant les radicaux aliphatiques linéaires ou ramifiés comprenant de 1 à 16 atomes de carbone, les radicaux aromatiques comprenant un ou plusieurs noyaux aromatiques sous forme condensée ou non, les radicaux aromatiques comprenant plusieurs noyaux aromatiques reliés entre eux par un radical aliphatique comprenant de 1 à 6 atomes de carbone ou un lien valenciel, et le radical B représente un groupement hydrocarboné choisi dans le groupe comprenant les radicaux aliphatiques linéaires ou ramifiés comprenant de 1 à 16 atomes de carbone, les radicaux comprenant un ou plusieurs noyaux aromatiques reliés aux atomes d'azote par un radical aliphatique, sous forme condensée ou non, ou reliés entre eux par un lien valenciel ou un radical aliphatique.

Avantageusement le radical A est choisi dans le groupe comprenant les radicaux polyméthyléniques non substitués ou substitués par des groupes alkyle, les radicaux benzoïque, alkyldiphénylénique.

On peut citer comme radicaux préférés : les radicaux hexaméthylène, pentaméthylène, tétraméthylène, méthyl-2 pentaméthylène, éthyl-2 pentaméthylène, métaphénylène, paraphénylène.

De manière analogue, le radical B est choisi dans le groupe comprenant les radicaux polyméthyléniques non substitués ou substitués par des groupes alkyles, comme par exemple les radiaux pentaméthylène, tétraméthylène, méthyl-2 pentaméthylène, éthyl-2 pentaméthylène.

En outre, le polymère obtenu peut comprendre plusieurs unités de récurrence de formule générale (I) se différenciant les unes des autres par au moins un des radicaux A et B. On peut citer notamment les copolymères comprenant des unités de récurrence comportant pour certaines comme radical A le radical paraphénylène et pour d'autres comme radical A le radical métaphénylène et/ou pour certaines comme radical B le radical hexaméthylène et pour d'autres comme radical B le radical alkylpentaméthylène.

Les polyamides produits peuvent être utilisés dans de nombreuses applications telles que film, fils, fibres ou compositions moulées comme cela est bien connu dans le domaine de la technique.

D'autres détails, caractéristiques et avantages de l'invention apparaîtront plus clairement au vu des exemples donnés ci-dessous uniquement à titre indicatif.

Le procédé de l'invention comprend deux étapes distinctes à savoir :
- une étape 1 qui consiste à fabriquer un monoamide-ester de formule générale (IV) par réaction d'une diamine sur un diester. Les exemples 1 à 11 illustreront cette étape 1, ainsi que le premier objet de l'invention,
- une étape 2 d'homopolymérisation du monoamide décrit ci-dessous. Cette étape et le deuxième objet de l'invention sont illustrés par les exemples 12 à 20,

### Exemples 1 à 6: préparation du composé

H₂N-(CH₂)₆ - NH - CO - (CH₂)₄ - CO - OCH₃ (V)

Dans un réacteur, on fond 1,03 mole d'hexaméthylène diamine. On ajoute 1 mole de diméthyladipate, le mélange est agité sous atmosphère non oxydante, telle que de l'azote. On ajoute ensuite le catalyseur.

Le mélange est maintenu à une température de 50°C. Après quelques minutes, un précipité apparaît. Au bout de quelques heures (2 à 10 heures) il y a prise en masse du milieu réactionnel.

Le produit obtenu est lavé à l'eau pour éliminer notamment les produits de départ. Le produit est ensuite séché sous vide et à froid.

Le produit obtenu est caractérisé par chromatographie d'exclusion stérique (SEC), et analyse RMN du solide du ¹³C.

Ces analyses confirment la formation d'une fonction amide et la conservation d'une fonction ester. Elles démontrent que le produit obtenu est de manière spécifique le produit de formule

H₂N-(-CH₂)₆- NH-CO-(CH₂)₄- CO-O-CH₃ (V)

Ce produit est obtenu avec un rendement pondéral de l'ordre de 90 %.

Dans le tableau I ci-dessous sont rassemblés les résultats de plusieurs exemples réalisés avec différents catalyseurs.

**Tableau I**

| Ex. | Catalyseur | % poids catalyseur | Température | Temps |
|---|---|---|---|---|
| 1 | NaOCH₃ | 1 | 50°C | 5 h |
| 2 | NaOCH₃ | 5 | 50°C | 5 h |
| 3 | NaOCH₃ | 10 | 50°C | 10 min |
| 4 | phénol | 5 | 50°C | 5 h |
| 5 | résorcinol | 10 | 50°C | 5 h |
| 6 | acide benzoïque | 5 | 50°C | 5 h |

### Exemples 7 à 9

Synthèse d'autres monoamide esters aliphatiques de formule :

H₂N - (CH₂)_{n₁} - NH - CO - (CH₂)_{n₂} - COOCH₃ (VI)

Cette synthèse est réalisée selon le mode opératoire décrit pour les exemples 1 à 6, également à une température de 50°C.

Les produits ont été obtenus avec un rendement de l'ordre de 90 %. La structure et la pureté de ces produits ont été confirmées par les analyses spectrales classiques et décrites précédemment.

Les différents monoamides synthétisés sont décrits dans le tableau II ci-dessous.

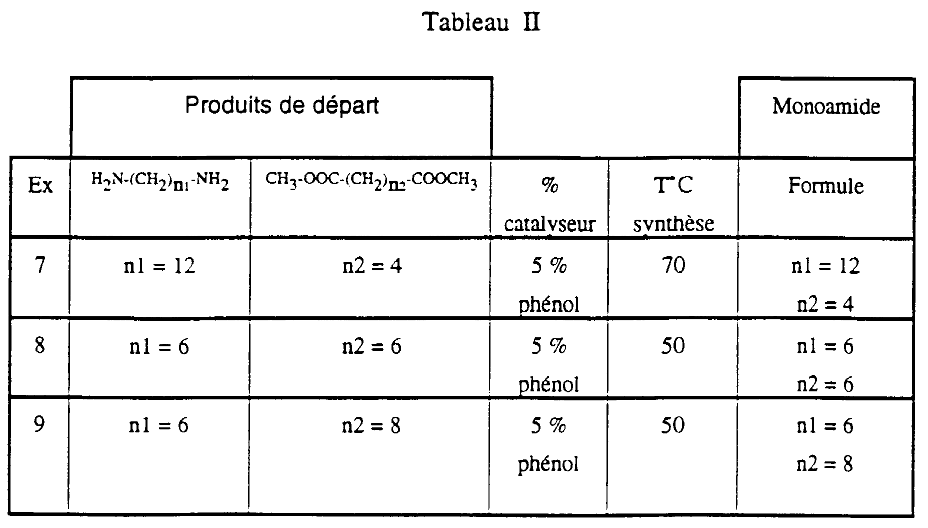

### Exemple 10 : synthèse du monoamide α-amino ω-ester de formule :

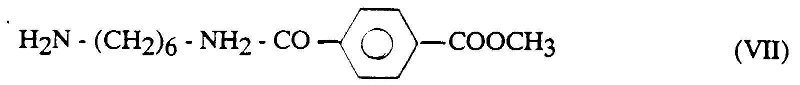

On introduit une mole de diméthyl téréphtalate et 1,03 mole d'hexaméthylène diamine. Le mélange maintenu sous atmosphère d'azote est fondu. Il est alors maintenu, sous agitation, à une température légèrement supérieure à la température de fusion, c'est-à-dire 160°C.

Après 30 minutes, et en absence de catalyseur, on observe la formation d'un précipité. La réaction est poursuivie jusqu'à prise en masse du milieu réactionnel. Après refroidissement et broyage, le produit est lavé à l'eau.

Les analyses spectrales montrent que le produit a la structure de la formule (VII).

### Exemple 11: synthèse du monoamide de formule :

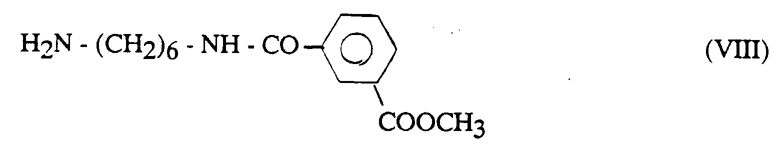

Ce monoamide ester est obtenu par mise en oeuvre du mode opératoire de l'exemple 10 avec remplacement du diméthyl téréphtalate par le diméthylisophtalate. La température de la réaction est de 90°C.

## Revendications

1. Procédé de préparation d'un monoamide α-amino ω-ester de formule générale (IV):
H₂N - B - NH - CO- A - COOR₁ (IV)
dans laquelle :
- B représente un reste de diamine et
- A représente un reste d'un diacide
- R₁ représente un groupement alkyle comprenant de 1 à 6 atomes de carbone,
caractérisé en ce qu'il consiste :
- à mélanger une diamine de formule
H₂N - B - NH₂ (II)
et un diester de formule
R₁OOC - A - COOR₁ (III)
dans un rapport molaire compris entre 0,8 et 1,2,
- à maintenir ce mélange en phase liquide à une température inférieure à la température de fusion ou de cristallisation du monoamide α-amino ω-ester formé, jusqu'à précipitation dudit monoamide α-amino ω-ester, et
- à récupérer le précipité formé.

2. Procédé selon la revendication 1, caractérisé en ce que le rapport molaire de la diamine au diester est compris entre 1 et 1,1.

3. Procédé selon la revendication 1 ou 2, caractérisé en ce que la réaction est poursuivie jusqu'à prise en masse du milieu.

4. Procédé selon l'une des revendications 1 à 3, caractérisé en ce que le mélange diamine/diester est fondu, la température de réaction étant supérieure à la température de fusion du mélange de 1 à 30°C.

5. Procédé selon l'une des revendications 1, 2 ou 3, caractérisé en ce que la diamine et le diester sont dissous dans un solvant, celui-ci n'étant pas solvant du monoamide α-amino ω-ester.

6. Procédé selon l'une des revendications précédentes, caractérisé en ce que la réaction est réalisée en présence d'un composé catalytiquement actif pour la réaction d'aminolyse.

7. Procédé selon la revendication 6, caractérisé en ce que le composé catalytiquement actif est présent dans un rapport pondéral compris entre 0,1 et 10 % par rapport à la masse du mélange.

8. Procédé selon l'une des revendications 6 ou 7, caractérisé en ce que le composé catalytiquement actif est un composé comprenant un groupement nucléophile et/ou un groupement électrophile.

9. Procédé selon la revendication 8, caractérisé en ce que le composé catalytiquement actif est un composé comprenant un groupement basique choisi dans le groupe comprenant les bases fortes, les alkyloxylates alcalins, les aryloxylates alcalins, et/ou un groupement acide choisi dans le groupe comprenant les acides forts,les acides et dérivés contenant du phosphore, les composés organo métalliques, les phénols, crésols et dérivés .

10. Procédé selon la revendication 8, caractérisé en ce que le composé catalytiquement actif est un composé comprenant un groupement N-hétérocycliques, choisi dans le groupe comprenant le pyrazole et ses dérivés, 8-hydroxyquinoléine.

11. Procédé selon l'une des revendications précédentes, caractérisé en ce que le radical A représente un groupement hydrocarboné aliphatique, aromatique, aromatique/aliphatique ou aliphatique/aromatique.

12. Procédé selon la revendication 11, caractérisé en ce que le radical A est choisi dans le groupe comprenant les radicaux aliphatiques linéaires ou ramifiés comprenant de 1 à 16 atomes de carbone, les radicaux aromatiques comprenant un ou plusieurs noyaux aromatiques sous forme condensée ou non, ou reliés entre eux par un lien valenciel ou un radical aliphatique comprenant de 1 à 6 atomes de carbone.

13. Procédé selon la revendication 12, caractérisé en ce que le radical A est choisi dans le groupe comprenant les radicaux polyméthyléniques linéaires ou substitués, para- ou méta-phénylénique.

14. Procédé selon l'une des revendications précédentes, caractérisé en ce que le radical B représente un groupement hydrocarboné choisi dans le groupe comprenant les radicaux aliphatiques linéaires ou ramifiés comprenant de 1 à 16 atomes de carbone, les radicaux comprenant un ou plusieurs noyaux aromatiques, reliés aux atomes d'azote par un radical aliphatique, sous forme condensée ou non, ou reliés entre eux par un lien valenciel ou un radical aliphatique.

15. Procédé selon la revendication 14, caractérisé en ce que le radical B représente un radical hexaméthylène, pentaméthylène, tétraméthylène, méthyl-2 pentaméthylène, éthyl-2 pentaméthylène.

## Patentansprüche

1. Verfahren zur Herstellung eines α-Amino-ω-ester-monoamides der allgemeinen Formel (IV)
H₂N-B-NH-CO-A-COOR₁ (IV)
in der
- B den Rest eines Diamins darstellt und
- A den Rest einer Disäure bedeutet,
- R₁ eine Gruppe Alkyl mit 1 bis 6 Kohlenstoffatomen ist,
dadurch gekennzeichnet, daß es darin besteht
- ein Diamin der Formel (II)
H₂N-B-NH₂ (II)
und einen Diester der Formel (III)
R₁OOC-A-COOR₁ (III)
in einem molaren Verhältnis zwischen 0,8 und 1,2 zu vermischen,
- diese Mischung in flüssiger Phase auf einer Temperatur von unterhalb der Schmelztemperatur oder der Kristallisationstemperatur des gebildeten α-Amino-ω-ester-monoamides bis zur Ausfällung des genannten α-Amino-ω-ester-monoamides zu halten, und
- den gebildeten Niederschlag zu gewinnen.

2. Verfahren nach Anspruch 1, dadurch gekennzeichnet, daß das molare Verhältnis des Diamins zum Ester zwischen 1 und 1,1 liegt.

3. Verfahren nach Anspruch 1 oder 2, dadurch gekennzeichnet, daß die Reaktion bis zur Agglomeration des Medium fortgesetzt wird.

4. Verfahren nach einem der Ansprüche 1 bis 3, dadurch gekennzeichnet, daß die Mischung Diamin/Diester geschmolzen wird, wobei die Temperatur der Reaktion 1 °C bis 30 °C höher liegt als die Schmelztemperatur der Mischung.

5. Verfahren nach einem der Ansprüche 1, 2 oder 3, dadurch gekennzeichnet, daß das Diamin und der Ester in einem Lösungsmittel gelöst werden, das kein Lösungsmittel für das α-Amino-ω-estermonoamid ist.

6. Verfahren nach einem der vorstehenden Ansprüche, dadurch gekennzeichnet, daß die Reaktion in Anwesenheit einer für die Reaktion der Aminolyse katalytisch aktiven Verbindung durchgeführt wird.

7. Verfahren nach Anspruch 6, dadurch gekennzeichnet, daß die katalytisch aktive Verbindung in einem Gewichtsverhältnis zwischen 0,1 % und 10 % anwesend ist, bezogen auf die Masse der Mischung.

8. Verfahren nach einem der Ansprüche 6 oder 7, dadurch gekennzeichnet, daß die katalytisch aktive Verbindung eine solche Verbindung ist, die eine nucleophile und/oder elektrophile Gruppe umfaßt.

9. Verfahren nach Anspruch 8, dadurch gekennzeichnet, daß die katalytisch aktive Verbindung eine solche Verbindung ist, die eine basische Gruppe umfaßt, gewählt aus der die starken Basen, die Alkalialkyloxylate, die Alkaliaryloxylate umfassenden Gruppe, und/oder die eine saure Gruppe umfaßt, gewählt aus der die starken Säuren, die Phosphor enthaltenden Säuren und Derivate, die Organometallverbindungen, die Phenole, die Kresole und Derivate umfassenden Gruppe.

10. Verfahren nach Anspruch 8, dadurch gekennzeichnet, daß die katalytisch aktive Verbindung eine solche Verbindung ist, die eine N-heterocyclische Gruppe umfaßt, gewählt aus der die Pyrazole und seine Derivate und 8-Hydroxychinolin umfassenden Gruppe.

11. Verfahren nach einem der vorstehenden Ansprüche, dadurch gekennzeichnet, daß der Rest A eine aliphatische, aromatische, aromatisch-aliphatische oder aliphatisch-aromatische Kohlenwasserstoffgruppe darstellt.

12. Verfahren nach Anspruch 11, dadurch gekennzeichnet, daß der Rest A aus der die geraden oder verzweigten aliphatischen Reste mit 1 bis 16 Kohlenstoffatomen, die aromatischen Reste mit einem oder mehreren aromatischen Kernen in kondensierter Form oder in nichtkondensierter Form oder untereinander verbunden durch eine Valenzbindung oder einen aliphatischen Rest mit 1 bis 6 Kohlenstoffatomen umfassenden Gruppe gewählt wird.

13. Verfahren nach Anspruch 12, dadurch gekennzeichnet, daß der Rest A aus der die geraden oder substituierten polymethylenischen, para- oder meta-phenylenischen Reste umfassenden Gruppe gewählt wird.

14. Verfahren nach einem der vorstehenden Ansprüche, dadurch gekennzeichnet, daß der Rest B eine Kohlenwasserstoffgruppe darstellt, gewählt aus der die geraden oder verzweigten aliphatischen Reste mit 1 bis 16 Kohlenstoffatomen, die Reste mit einem oder mehreren aromatischen Kernen, verbunden mit Stickstoffatomen durch einen aliphatischen Rest in kondensierter Form oder in nichtkondensierter Form oder untereinander verbunden durch eine Valenzbindung oder einen aliphatischen Rest umfassenden Gruppe.

15. Verfahren nach Anspruch 14, dadurch gekennzeichnet, daß der Rest B einen Rest Hexamethylen, Pentamethylen, Tetramethylen, 2-Methyl-pentamethylen oder 2-Ethyl-pentamethylen darstellt.

## Claims

1. Process for the preparation of an α-amino ω-ester monoamide of general formula (IV):
H₂N-B-NH-CO-A-COOR₁ (IV)
in which:
- B denotes a diamine residue and
- A denotes a residue of a diacid
- R₁ denotes an alkyl group containing from 1 to 6 carbon atoms,
characterized in that it consists:
- in mixing a diamine of formula:
H₂N-B-NH₂ (II)
and a diester of formula:
R₁OOC-A-COOR₁ (III)
in a molar ratio of between 0.8 and 1.2,
- in keeping this mixture in liquid phase at a temperature below the melting or crystallization temperature of the α-amino ω-ester monoamide formed, until the precipitation of the said α-amino ω-ester monoamide, and
- in recovering the precipitate formed.

2. Process according to Claim 1, characterized in that the molar ratio of the diamine to the diester is between 1 and 1.1.

3. Process according to Claim 1 or 2, characterized in that the reaction is continued until the mixture sets solid.

4. Process according to one of Claims 1 to 3, characterized in that the diamine/diester mixture is melted, the reaction temperature being from 1 to 30°C higher than the melting temperature of the mixture.

5. Process according to one of Claims 1, 2 or 3, characterized in that the diamine and the diester are dissolved in a solvent, the latter not being a solvent for the α-amino ω-ester monoamide.

6. Process according to one of the preceding claims, characterized in that the reaction is carried out in the presence of a compound which is catalytically active in the aminolysis reaction.

7. Process according to Claim 6, characterized in that the catalytically active compound is present in a weight ratio of between 0.1 and 10 % relative to the mass of the mixture.

8. Process according to either of Claims 6 and 7, characterized in that the catalytically active compound is a compound including a nucleophilic group and/or an electrophilic group.

9. Process according to Claim 8, characterized in that the catalytically active compound is a compound including a basic group chosen from the group including strong bases, alkali metal alkoxides, alkali metal aryloxides and/or an acid group chosen from the group including strong acids, acids and derivatives containing phosphorus, organometallic compounds, phenols, cresols and derivatives.

10. Process according to Claim 8, characterized in that the catalytically active compound is a compound including an N-heterocyclic group, chosen from the group including pyrazole and its derivatives and 8-hydroxyquinoline.

11. Process according to one of the preceding claims, characterized in that the radical A denotes an aliphatic, aromatic, aromatic/aliphatic or aliphatic/aromatic hydrocarbon group.

12. Process according to Claim 11, characterized in that the radical A is chosen from the group including linear or branched aliphatic radicals containing from 1 to 16 carbon atoms, aromatic radicals including one or a number of aromatic nuclei in condensed or uncondensed form or joined to one another by a valency bond or an aliphatic radical containing from 1 to 6 carbon atoms.

13. Process according to Claim 12, characterized in that the radical A is chosen from the group including linear or substituted polymethylene radicals and para- or meta-phenylene radicals.

14. Process according to one of the preceding claims, characterized in that the radical B denotes a hydrocarbon group chosen from the group including linear or branched aliphatic radicals containing from 1 to 16 carbon atoms, and radicals including one or a number of aromatic nuclei joined to nitrogen atoms by an aliphatic radical, in condensed or uncondensed form, or joined to one another by a valency bond or an aliphatic radical.

15. Process according to Claim 14, characterized in that the radical B denotes a hexamethylene, pentamethylene, tetramethylene, 2-methylpentamethylene or 2-ethylpentamethylene radical.
